# EUROPEAN PATENT APPLICATION

(11) **EP 3 115 450 A1**
(43) Date of publication of application: **11.01.2017**
(21) Application number: 15757861.8
(22) Date of filing: 02.03.2015
(51) Int. Cl.: C12N 1/00, C12M 1/00, C12M 1/02, C12M 3/00

(54) **CELL CULTURE METHOD AND CELL CULTURE DEVICE**

(30) Priority: 07.03.2014 JP 2014044641
(71) Applicant: Toyo Seikan Group Holdings, Ltd., Shinagawa-ku Tokyo 141-8627 (JP)
(72) Inventor: HATA, Norihiko, Tokyo 141-8627 (JP)
(74) Representative: Liebetanz, Michael
(86) International application number: PCT/JP2015/001080
(87) International publication number: WO 2015/133116

(57) **Abstract**

When cell culture is conducted by supplying a culture medium together with cells to a container 1, an upward movement step in which the container 1 is moved upward to a position higher than the initial position and a downward movement step in which the container 1 that has been moved upward is returned to the initial position, and vibration is applied to the container 1 that has been returned to the original positon are repeated, whereby a content liquid in the container 1 is stirred. As a result, a good culture environment is maintained by conducting stirring such that distribution of cells and concentration of components of a culture medium in a content liquid become uniform, while reducing damage on cells by shear stress, and adherence of cells to the inner wall or excessive agglomeration is suppressed by stirring, whereby proliferation of cells can be promoted.

## Description

### Technical Field

The present invention relates to a cell culture method and a cell culture device in which cells, tissues, microorganisms or the like are cultured in an artificial environment.

### Background Art

In recent years, in the fields of production of pharmaceuticals, gene therapy, regenerative medicine, immunotherapy or the like, it has been required to culture cells, tissues, microorganisms or the like efficiently in a large amount in an artificial environment, and various cell culture devices are known. For example, Patent Document 1 proposes a cell culture device in which cell culture is conducted by connecting, to a cell culture bag, a culture medium bag, a cultured cell collection bag, a connection tube or the like.

In this type of cell culture device, in order to promote proliferation of cells, it is desired that a good culture environment be maintained by conducting stirring such that distribution of cells and concentration of components of a culture medium in a content liquid in a cell culture bag become uniform. In addition, there are problems that cells are adhered to the inner wall or agglomerated excessively, or the like, resulting in lowering of proliferation ratio of cells.

Therefore, Patent Document 2 proposes a rotating culture device in which a cell culture bag is attached to a rotating plate that is inclined at an appropriate angle, and a content liquid is stirred by the rotation of such rotating plate. Patent Document 3 proposes a swinging culture device in which a cell culture bag is attached to a swinging plate that can be moved upward and downward with respect to the X-axis and the Y-axis, and a content liquid is stirred by swinging of such swinging plate.

### Related Art Documents

### Patent Documents

- Patent Document 1:: JP-A-S63-248382
- Patent Document 2:: Utility Model Application Unexamined Publication No. S63-155399
- Patent Document 3:: JP-A-S64-18433

### Disclosure of the Invention

### Problems to be Solved by the Invention

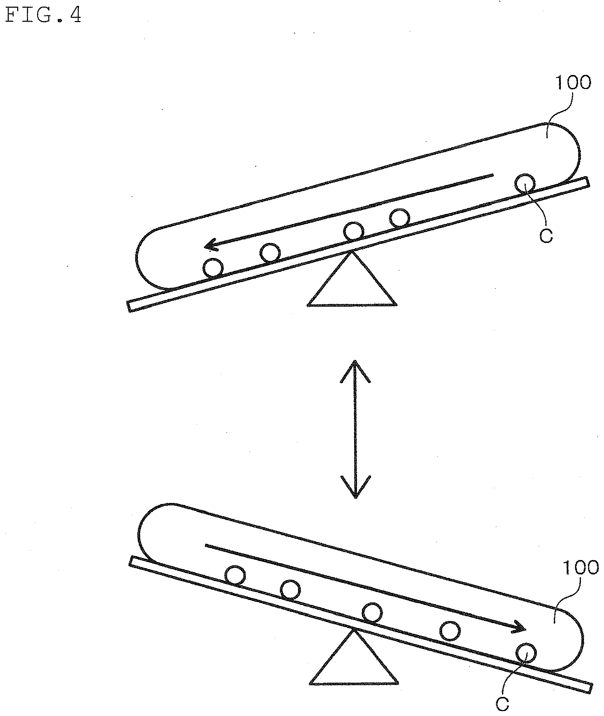

However, as in the case of a device disclosed in Patent Document 2 or Patent Document 3, there is a concern that, if a content liquid in the cell culture bag is stirred by rotation or swinging, as shown in Fig. 4, for example, cells C in the content liquid are rolled down for a long distance on the culture surface of the cell culture bag 100, and as a result, cells are damaged by shear stress.

The present invention has been made in view of the above-mentioned circumstances, and is aimed at providing a cell culture method and a cell culture device in which a good culture environment is maintained by conducting stirring such that distribution of cells and concentration of components of a culture medium become uniform, while reducing damage on cells by shear stress, and adherence of cells to the inner wall or excessive agglomeration is suppressed by stirring, whereby proliferation of cells can be promoted.

### Means for Solving the Problems

The cell culture method according to the present invention is a cell culture method that supplies a culture medium to a culture container together with cells to conduct cell culture, wherein
an upward movement step in which the culture container is moved upward to a position higher than an initial position and
a downward movement step in which the culture container that has been moved upward is returned to the initial position, and vibration is applied to the culture container that has been returned to the initial position
are repeated,
whereby a content liquid in the culture container is stirred.

The cell culture device according to the present invention is a cell culture device that supplies a culture medium together with cells to a culture container to conduct cell culture comprising:
a holding part having a flat plate-shaped holding plate in which a turning shaft provided on one end thereof is pivotably supported on a base, and
a cam mechanism in which the holding plate is used as a follower, wherein
the culture container is held in the holding part, and
the holding plate is turned upward and downward repeatedly around the turning shaft by the cam mechanism, and vibration generated when the holding plate is turned downward and contacts with the base is applied to the culture container, whereby a content liquid in the culture container is stirred.

### Advantageous Effects of the Invention

According to the present invention, it is possible to maintain a good culture environment by conducting stirring such that distribution of cells and concentration of components in a culture medium becomes uniform, while reducing damage on cells by shear stress, and further, adherence of cells to the inner wall or excessive agglomeration is suppressed by stirring, whereby proliferation of cells can be promoted.

### Brief Description of the Drawings

Fig. 1 is an explanatory view showing an outline of the embodiment of the present invention;
Fig. 2 is an explanatory view showing an outline of a modification example of the embodiment of the present invention;
Fig. 3 is an explanatory view showing an example of a driving mechanism that turns the holding plate upward and downward in other modification examples of the embodiment of the present invention; and
Fig. 4 is an explanatory view showing an outline of conventional technology.

### Mode for Carrying out the Invention

Hereinbelow, an explanation will be made on preferred embodiments of the cell culture method and the cell culture device of the present invention with reference to the drawings.

In this embodiment, to a container 1 as a culture container, cells to be cultured and a culture medium (including a culture liquid or a culture medium) for culturing these cells are supplied as a content liquid.

In this embodiment, the container 1 is made of a flexible material and has a shape of a bag. It can conduct cell culture in a closed (sealed) system. Further, it has permeability to a gas such as oxygen or carbon dioxide that is required when used in a CO₂ incubator. Further, in order to allow the content to be visible, part or all of the container 1 is transparent. As specific examples of a flexible material that satisfies the conditions as the container 1, polyolefin, ethylene-vinyl acetate copolymers, styrene-based elastomers, polyester-based thermoplastic elastomers, silicone-based thermoplastic elastomers, silicone rubber or the like can be given.

Not particularly shown, normally the shape of the container 1 is rectangular. To the container 1, a supply tube for supplying cells or a culture medium from the outside and a collection tube for collecting cells or a culture medium from the container 1 are connected. These tubes may be connected to opposing two sides on the shorter side of the container 1 such that one tube is connected to one side, or two tubes may be connected to one side on the shorter side or one side on the longer side.

The number of tubes connected to the container 1 is not limited to two. In addition to these two tubes, a tube, etc. for taking out a sample may be connected, or supply and collection may be conducted by one tube. The shape of the container 1 may be elliptical, circular or the like, and various shapes can be given according to need.

The material of the tube to be connected to the container 1 may be appropriately selected according to the environment of use. When the tube is used in a CO₂ incubator, it is particularly desirable to use one having excellent gas permeability for oxygen and carbon dioxide. For example, silicone rubber, soft vinyl chloride resins, polybutadiene resins, ethylene-vinyl acetate copolymers, chlorinated polyethylene resins, polyurethane-based thermoplastic elastomers, polyester-based thermoplastic elastomers, silicone-based thermoplastic elastomers, styrene-based elastomers or the like can be used. As the styrene-based elastomer, SBS (styrene-butadiene-styrene), SIS (styrene-isoprene-styrene), SEBS (styrene-ethylene-butylene-styrene), SEPS (styrene-ethylene-propylene-styrene) or the like can be used.

In this embodiment, when cell culture is conducted by using such container 1 as a culture container, an upward movement step in which the container 1 is moved upward to a position higher than the initial position and a downward movement step in which the container 1 is returned to the initial position and vibration is applied to the container 1 that has been returned to the initial position are repeated, whereby a content liquid in the container 1 is stirred.

When repeating the upward movement step and the downward movement step, in order to allow stirring of the content liquid in the container 1 to be conducted without fail, according to need, the upward movement step is conducted at an interval (i.e. after the lapse of a prescribed period of time) after the completion of the downward movement step.

Due to such a configuration, in addition to stirring of a content liquid by the upward and downward movements of the container 1, due to the vibration applied to the container 1 in the downward movement step, cells are diffused in the content liquid as if they fly up. As a result, the content liquid in the container 1 is efficiently stirred in the upward and downward directions, and while reducing the shear stress when cells roll down on the culture surface of the container 1 and minimizing damage on cells, distribution of cells and concentration of components of a culture medium in the content liquid in the container 1 is allowed to be uniform, whereby a good culture environment can be maintained. In addition, adhesion of cells to the inner wall of the container or excessive agglomeration of cells can be suppressed, whereby proliferation of cells can be promoted. Further, when cells to be cultured are adherent cells, by vibration applied to the container 1 in the downward movement step, it is possible to promote peeling off of cells from the culture surface of the container 1.

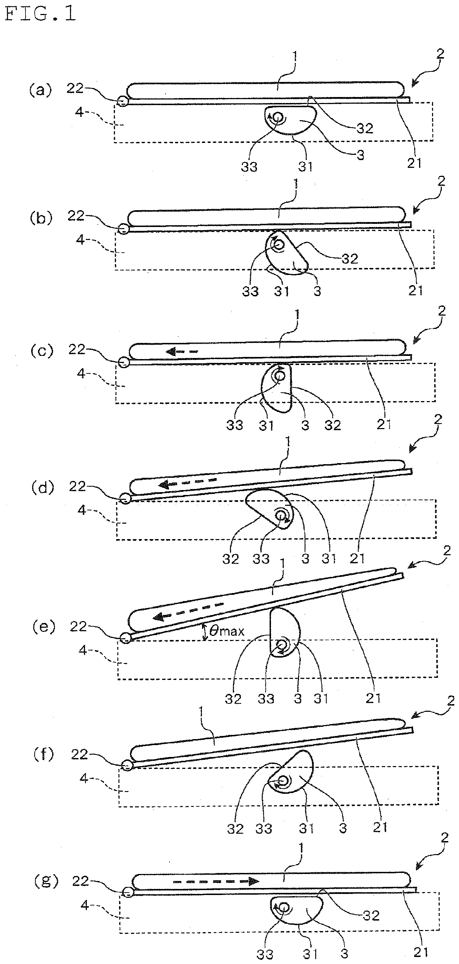

In order to stir the content liquid in the container 1 as mentioned above, as shown in Fig. 1, for example, it is preferable to use a device comprising: a holding part 2 having a flat plate-shaped holding plate 21 in which a turning shaft 22 that is rotatably provided on one end thereof is pivotably supported on a base 4, and a cam mechanism in which the holding plate 21 is used as a follower, wherein the container 1 is held in a holding part 2, and the holding plate 21 is turned upward and downward repeatedly around the turning shaft 22 by the cam mechanism, and vibration generated when the holding plate 21 is turned downward and contacts with the base 4 is applied to the container 1, whereby a content liquid in the container 1 is stirred.

In such a device, in the upward movement step, the container 1 held in the holding part 2 starts to incline while moving upward from the initial position as the holding plate 21 is turned upward by the cam mechanism. As a result, while the container 1 that is formed of a flexible material is flexuously deformed, the content liquid flows to the turning shaft 22 such that it is gathered on one side (see Figs. 1(a) to (e)). In the downward movement step, the holding plate 21, after reaching the maximum inclination angle θₘₐₓ, is turned downward, and as a result, the container 1 is returned to the initial position (see Figs. 1(f) and (g)). At this time, swing back occurs in the content liquid in the container 1, and at the same time, the cells are diffused as if they fly in the content liquid by vibration generated by impact occurred when the holding plate 21 contacts with the base 4, whereby the content liquid in the container 1 is stirred.

When the content liquid in the container 1 is stirred in this way, in order to minimize the damage on the cells to allow the content liquid in the container 1 to be stirred more efficiently, it is preferred that the holding plate 21 that is supported horizontally on the base 4 be turned upward slowly, and after the holding plate 21 reaches the maximum inclination angle θₘₐₓ, the holding plate 21 be turned moved downward by falling, so that vibration is applied to the container 1 by impact that occurs when the falling holding plate 21 is received on the base 4. Although not particularly shown, when the vibration generated by impact occurred when the holding plate 21 contacts with the base 4 is applied to the container 1, it is possible to provide a convex part that contacts with the holding plate 21 in the base 4 at a position that is preferable for applying vibration to the container 1 in respect of efficient stirring of the content liquid in the container 1.

Further, the holding plate 21 can be turned downward by falling, as follows, for example. A cam mechanism is constituted by a plate cam 3 having a first cam surface 31 having a circular contour and a second cam surface 32 having a linear contour, the curvature of the cam surface 31 and 32, the position of the driving shaft 33 which drives the plate cam 3 and the rotational speed of the driving shaft 33 are appropriately set, and the holding plate 21 is turned upward by the first cam surface 31 and the plate cam 3 is moved away from the holding plate 21 when the plate cam 3 is switched to the second cam surface 32 (see Fig. 1(f)), whereby the holding plate 21 can be turned downward by falling.

As mentioned above, in many cases, the shape of the container 1 is normally rectangular, and the shape of the holding plate 21 is designed in accordance with the shape of the container 1. When the holding plate 21 is formed to be rectangular, the one end at which the turning shaft 22 is provided may be on the shorter side or may be on the longer side.

The present embodiment as mentioned above can be applied to a case where floating cells or adherent cells are cultured. As mentioned above, when applying to the culture of adherent cells, it is possible to promote peeling of cells from the culture surface of the container 1. By subjecting the content liquid in the container 1 to a prescribed treatment (e.g. addition of a protein decomposing enzyme such as trypsin) according to need, it can be used also as means for peeling adherent cells from the culture surface.

The present invention is described hereinabove with reference to preferred embodiments. The present invention is not restricted to the above-mentioned embodiments, and it is needless to say that various modifications are possible within the scope of the present invention.

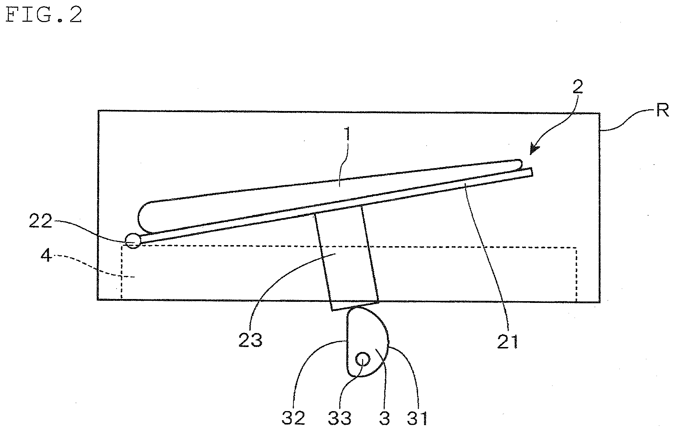

For example, in the above-mentioned embodiment, an example is shown in which the plate cam 3 abuts the lower surface of the holding plate 21 to allow the holding plate 21 to turn upward and downward. The specific embodiment of the cam mechanism is not limited thereto. For example, when the container 1 is accommodated together with the holding part 2 in a culture chamber which is kept at a prescribed temperature to conduct cell culture, if it is hard to secure an installation space of the cam mechanism in the culture chamber, as shown in Fig. 2, a follower member 23 is provided on the lower surface of the holding plate 21, thereby to allow the plate cam 3 that is provided outside of the culture chamber R to abut the follower member 23 and to allow the holding plate 21 to turn upward and downward.

In the above-mentioned embodiment, an example is shown in which the container 1 made of a flexible material and has a shape of a bag is used as the culture container. However, the culture container may be an integrally-molded bag formed by blow molding, or the like. The specific feature of the container 1 is not particularly restricted as long as it can allow the content liquid to flow and to be stirred by the upward and downward movement of the container 1.

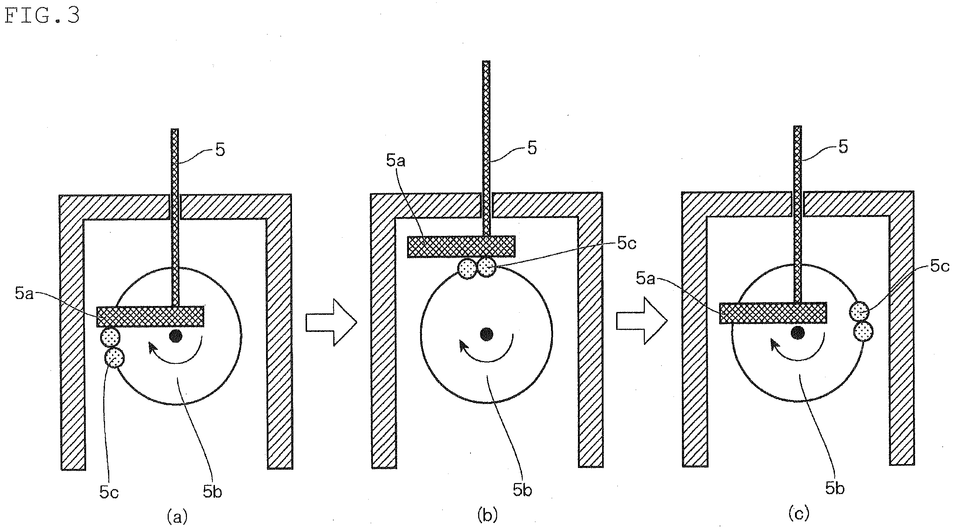

In the above-mentioned embodiment, an example is given in which the holding plate 21 is turned upward and downward by the cam mechanism using the holding plate 21 as the follower. The specific embodiment in which the holding plate 21 is turned upward and downward is not limited thereto. For example, other power transmission mechanisms such as a crank mechanism may be used. By the driving mechanism shown in Fig. 3, the holding plate 21 may be turned upward and downward.

The driving mechanism shown in Fig. 3 is provided with a rotating plate 5b in which a protrusion 5c is provided on the periphery, a receiving plate 5a in which one end thereof extends to the periphery of the rotating plate 5b and the other end does not reach the periphery of the rotating plate 5b, and a push-up member 5 in which the receiving plate 5a is provided at a base end thereof. When the rotating plate 5b is rotated, the receiving plate 5a is pushed up by the protrusion 5c and the push-up member 5 is moved upward (see Fig. 3(b)). Subsequently, when the protrusion 5c is removed from the receiving plate 5a, the push-up member 5 is moved downward by its own weight (see Fig. 3(c)). In such a driving mechanism, the push-up member 5 that repeatedly conducts upward and downward movements by rotation of the rotating plate 5b in a fixed direction contacts the bottom surface of the holding plate 21, whereby the holding plate 21 can be turned upward and downward repeatedly. Then, when the push-up member 5 is moved downward, it falls due to its own weight, and by applying to the container 1 vibration generated when the holding plate 21 that falls together with the falling of the push-up member 5 contacts with the base 4, the content liquid can be stirred.

Also in this embodiment, by appropriately set the angular velocity at which the holding plate 21 is turned upward and downward, as well as the maximum inclining angle θₘₐₓ of the holding plate 21, the shear stress when cells roll down the culture surface of the container 1 can be reduced, and the content liquid in the container 1 can be stirred efficiently

Further, in the above-mentioned embodiment, an example is shown in which vibration to be applied to the container 1 is generated by allowing the holding plate 21 to be turned downward by falling and to be received on the base 4. Specific embodiment in which vibration is applied to the container 1 is not limited thereto. For example, vibration may be generated when the holding plate 21 contacts with the base 4 by appropriately adjusting the angular velocity at which the holding plate 21 is turned moved downwardly.

In the above-mentioned embodiment, an example is shown in which the flat plate-shaped holding plate 21, in which the turning shaft 22 provided on the one end thereof is pivotably supported on the base 4, is turned upward and downward to conduct the upward movement step and the downward movement step. The specific feature in which the upward movement step and the downward movement step are conducted is not limited thereto. For example, although not particularly shown, by supporting, in a loosely fitted condition, the four corners of a square-shaped holding plate to a vertically-arranged shaft, the culture container is retained in this holding plate, and the upward movement step and the downward movement step are conducted by an appropriate power transmission mechanism.

The features of the holding part 2 are not limited to the features explained in the above-mentioned embodiment. As in the case of the holding part provided in the device that was previously proposed by the applicant in Japanese Patent Application No. 2013-261159 or Japanese Patent Application No. 2013-261160, the holding plate 2 may be configured such that it has a bottom plate that supports the center part in the width direction of the container 1 and side plates that are provided turnably along the both ends of the bottom plate.

### Industrial Applicability

The present invention can be preferably utilized as a technology of culturing cells in regenerative medical treatment or production of antibody drugs.

### Explanation of Numerical Symbols

- 1.: Container (culture container)
- 2.: Holding part
- 21.: Holding plate
- 22.: Turning shaft
- 3.: Plate cam
- 4.: Base
- 5.: Push-up member
- 5a.: Receiving plate
- 5b.: Rotating plate
- 5c.: Protrusion

## Claims

1. A cell culture method that supplies a culture medium to a culture container together with cells to conduct cell culture, wherein
an upward movement step in which the culture container is moved upward to a position higher than an initial position and
a downward movement step in which the culture container that has been moved upward is returned to the initial position, and vibration is applied to the culture container that has been returned to the initial position
are repeated,
whereby a content liquid in the culture container is stirred.

2. The cell culture method according to claim 1, wherein the content liquid in the culture container is gathered on one side by inclining the culture container while moving it upward, whereby swing back is caused in the content liquid in the culture container when the culture container is returned to the initial position.

3. The cell culture method according to claim 1 or 2, wherein
the culture container is held in a holding part having a flat plate-shaped holding plate in which a turning shaft provided on one end thereof is pivotably supported on a base,
the holding plate is turned upward around the turning shaft, thereby allowing the culture container to be moved upward to a position higher than the initial position while being inclined, and
the holding plate is turned downward around the turning shaft and the holding plate contacts with the base when the culture container is returned to the initial position, whereby vibration applied to the culture container is generated.

4. The cell culture method according to claim 3, wherein the holding plate is turned downward by falling and received on the base, whereby vibration applied to the culture container is generated.

5. The cell culture method according to claim 3 or 4, wherein the holding plate is turned upward and downward by a cam mechanism using the holding plate as a follower.

6. The cell culture method according to claim 3 or 4, wherein the holding plate is turned upward and downward by a driving mechanism including a rotating plate having protrusion provided on the periphery, a receiving plate of which the one end extends to the periphery of the rotating plate but the other end does not reach the periphery of the rotating plate, and a push-up member in which the receiving plate is provided at a base end thereof, and
when the rotating plate is rotated, the receiving plate is pushed up by the protrusion, thereby to allow the push-up member to be moved upward, and then, when the protrusion are removed from the receiving plate, the push-up member is moved downwardly by its own weight.

7. The cell culture method according to any one of claims 1 to 6, wherein the culture container is formed of a flexible material.

8. A cell culture device that supplies a culture medium together with cells to a culture container to conduct cell culture comprising:
a holding part having a flat plate-shaped holding plate in which a turning shaft provided on one end thereof is pivotably supported on a base, and
a cam mechanism in which the holding plate is used as a follower, wherein
the culture container is held in the holding part, and
the holding plate is turned upward and downward repeatedly around the turning shaft by the cam mechanism, and vibration generated when the holding plate is turned downward and contacts with the base is applied to the culture container, whereby a content liquid in the culture container is stirred.

9. A cell culture device that supplies a culture medium together with cells to a culture container to conduct cell culture comprising:
a holding part having a flat plate-shaped holding plate in which a turning shaft provided on one end thereof is pivotably supported on a base, and
a driving mechanism having a rotating plate having protrusion provided on the periphery, a receiving plate of which the one end extends to the periphery of the rotating plate but the other end does not reach the periphery of the rotating plate, and a push-up member in which the receiving plate is provided at a base end thereof, and when the rotating plate is rotated, the receiving plate is pushed up by the protrusion, thereby to allow the push-up member to be moved upward, and then, when the protrusion is removed from the receiving plate, the push-up member is moved downwardly by its own weight, wherein
the culture container is held in the holding part, and
the push-up member that repeats the upward and downward movements by the rotation of the rotating plate in one direction abuts a bottom surface of the holding plate, and turns the holding plate upward and downward repeatedly, and vibration that is generated when the holding plate is turned downward and contacts with the base is applied to the culture container, whereby a content liquid in the culture container is stirred.
